# EUROPÄISCHE PATENTSCHRIFT

(11) **EP 0 792 129 B1**
(45) Veröffentlichungstag und Bekanntmachung des Hinweises auf die Patenterteilung: **13.02.2002**
(21) Anmeldenummer: 95936972.9
(22) Anmeldetag: 20.11.1995
(51) Int. Cl.: A61F 2/36

(54) **MODULARE GELENKPROTHESE**
MODULAR JOINT PROSTHESIS
PROTHESE ARTICULAIRE MODULAIRE

(30) Priorität: 19.11.1994 DE 4442204
(43) Veröffentlichungstag der Anmeldung: 03.09.1997
(73) Patentinhaber: BIOMET Merck Deutschland GmbH, 14167 Berlin (DE)
(72) Erfinder: LOB, Günter, D-81377 München (DE); FISCHER, Hans-Joachim, D-12277 Berlin (DE); STEÜR, Gerd, D-10551 Berlin (DE); KRANZ, Curt, D-10825 Berlin (DE)
(74) Vertreter: Gross, Felix, Dr.
(86) Internationale Anmeldenummer: DE9501655
(87) Internationale Veröffentlichungsnummer: WO9615739

(56) Entgegenhaltungen:
- EP-A- 0 000 549
- EP-A- 0 190 981
- EP-A- 0 243 298
- EP-A- 0 290 735
- EP-A- 0 356 376
- EP-A- 0 462 357
- EP-A- 0 465 436
- EP-A- 0 611 225
- EP-A- 0 611 558
- EP-A- 0 623 321
- DE-U- 9 401 529
- DE-U- 9 418 963

## Beschreibung

Die Erfindung betrifft eine modulare Gelenkprothese der im Oberbegriff des Anspruchs 1 angegebenen Art, wie sie beispielsweise aus der EP-A-0 190 981 als bekannt hervorgeht.

Gelenkprothesen werden in unterschiedlichsten Formen und Größen insbesondere als Hüftgelenkprothesen hergestellt, um eine gute Anpassung an die anatomischen Bedingungen des jeweiligen Patienten zu schaffen.

Durch mehrteilige Ausführung mit kraftschlüssiger Verbindung der entsprechenden Einzelteile im proximalen Bereich kann die Anpassung optimal erfolgen. Dabei ist gleichzeitig ein Positionierung des Gelenkkopfes unabhängig vom Schaftdurchmesser möglich.

Aus der EP-B1 0 243 298 ist ein Bausatz für eine Schaftprothese bekannt, der ein mit einer Gelenkkugel versehbares Kopfteil, ein im Knochen verankerbares Endteil und ein zwischen beiden positionierbares Zwischenteil enthält. Alle Teile weisen konische Bohrungen bzw. dazu passende Zapfen auf, wodurch die Prothese durch Herstellen konischer Steckverbindungen zusammengesetzt werden kann. Kopf- und Zwischenteil weisen jeweils eine axiale Durchgangsbohrung auf.

Beim Zusammenfügen der Einzelteile sind die entsprechenden Bohrungen axial in Richtung des Schaftes ausgerichtet. Die Einzelteile der Prothese werden unter Verwendung eines eine Kraft in axialer Richtung übertragenden Zugankers zusammengefügt, welcher sowohl das Kopfteil als auch nachfolgende Schaftteile durchdringt und in die Gewindebohrung des Endteils einschraubar ist. Dadurch werden das Kopfteil, oder das Zwischenteil und das Endteil fest zusammengezogen, so daß eine Lockerung der einzelnen Prothesenteile durch die mechanische Belastung bei Benutzung nicht zu befürchten ist.

Die vorstehend beschriebene Lösung weist den Nachteil auf, daß bei der vorbekannten Hüftgelenkprothese aufgrund der Gestaltung des Kopfteils bzw. der zweiteiligen Ausbildung des Schaftteils unter Verwendung eines zusätzlichen Zwischenteils ein universelles, d.h. eine für die Mehrzahl der zu erwartenden Krankheitsfälle einsetzbare Ausführung nicht erzielbar ist.

Ausgehend vom Stand der Technik liegt der Erfindung die Aufgabe zugrunde, eine modulare Hüftgelenkprothese der eingangs genannten Gattung zu schaffen, welche durch die Ausbildung seiner Einzelteile die Möglichkeit zur Herstellung einer universell verwendbaren Prothese bietet und welche keinem frühzeitigen Verschleiß unterliegt.

Diese Aufgabe wird mit den kennzeichnenden Merkmalen des Anspruchs 1 gelöst.

Die Erfindung schließt die Erkenntnis ein, daß durch die zweiteilige Ausbildung einer Hüftgelenkprothese günstige Bedingungen für einen universellen Einsatz vorhanden sind, wenn die sich ergebende Bauform unabhängig von der Art der Montage an die Form einer heutigen Standard-Prothese angepaßt ist. Dies wurde mit den erfindungsgemäßen Maßnahmen unabhängig von der Ausrichtung des Schaftendes in Bezug auf das Kopfteil erreicht. Hierbei ist insbesondere dafür gesorgt, daß die Steckverbindung für die Verbindung der Schaftteile in einem Bereich gelegen ist, der bei Anpassung an den geräumten Markraum für dessen Aufnahme keine Ausbuchtungen oder Erweiterungen benötigt. Mit den erfindungsgemäßen Maßnahmen läßt sich eine Schaftprothese erzeugen, die bei üblichen Abmessungen (und insbesondere unter präziser Anpassung an den geräumten Knocheninnenraum) eine patientenindividuelle Gestaltung ermöglicht. Dies läßt sich bei einer Mindestzahl von vorrätig zu haltenden Baugruppen und damit kostengünstig erzielen.

Dies ist auch gleichzeitig eine Voraussetzung dafür, daß aus einer rechtsseitig implantierbaren Prothese lediglich durch eine 180°-Schwenkbewegung des Prothesenkopfteils um die Längsachse der Steckverbindung eine linksseitig implantierbare Hüftgelenkprothese hergestellt werden kann, wobei auch alle Zwischenstellungen möglich sind. Dadurch, daß sich der Schaftbereich unterhalb der Verbindungsstelle konisch erweitert, ist auch hier bereits für eine sichere Krafteinleitung in den Knochen gesorgt, so daß der Verbindungsbereich entlastet ist.

Mit den erfindungsgemäßen Maßnahmen lassen sich auch extrem lange Prothesen in unterschiedlichen Variationen zusammenfügen, wobei durch die Möglichkeit der optimalen Formeinstellung durch Rotation im Verbindungsbereich der optimale Sitz einer jeden Prothesenzusammenstellung in anatomisch korrekter Form sichergestellt ist. Mit den Mitteln der Erfindung läßt sich mit einer Bausatzprothese im Ergebnis nahezu die individuelle Paßgenauigkeit einer patientenindividuell gefertigten Prothese erzielen.

Entsprechend der Erfindung ist die für eine zementfreie Implantation vorgesehene Hüftgelenkprothese aus einem Kopfteil und einem Schaftteil zusammensetzbar. Dabei ist am proximalen Ende des Schaftteils ein konischer Zapfen und am distalen Ende des Kopfteils eine Ausnehmung mit entsprechenden Abmaßen vorgesehen, welche nach erfolgter Montage der Gelenkprothese eine Steckverbindung bilden. Das Kopfteil ist in eine Basis und einen zum Tragen der Gelenkkugel vorgesehenen Anschlußzapfen gegliedert. Das Querschnittsprofil der Basis des Kopfteils ist im wesentlichen elliptisch ausgebildet, wobei die große Halbachse der Querschnitts-Ellipse nach distal kontinuierlich abnimmt.

An der dorsalen bzw. frontalen Seitenfläche des Kopfteils ist jeweils eine Profilierung aus sich axial erstreckenden, verrundete Außenkanten aufweisende Rippen vorgesehen. Dadurch wird in vorteilhafter Weise eine große Oberfläche geschaffen, die eine Fixation der Prothese durch Einwachsen von Knochenmaterial begünstigt.

Die Basis des Kopfteils weist bevorzugt ein bei Ansicht von dorsal bzw. frontal ein sich in proximaler Richtung erweiterndes Längsschnittprofil auf, das an seiner lateralen Seite durch zwei, einen stumpfen Winkel einschließende Geraden und an seiner medialen Seite durch ein konkaves Bogenstück begrenzt ist. Dabei setzt sich die distal befindliche Gerade des Geradenpaares und das Bogenstück jeweils in einer Geraden fort, welche das sich nach distal konisch verjüngende Längsschnittsprofil eines sich unterhalb des Einsteckkonus befindlichen proximalen Abschnitts des Schaftteils begrenzen.

Die Geraden des Geradenpaares des Kopfteil-Profils weisen eine unterschiedliche Länge und jeweils eine Neigung in Richtung der Mittelachse des Kopfteils auf, wobei sich die kürzere Gerade am distalen Ende des Kopfteils befindet.

Nach einer günstigen Weiterbildung der Erfindung ist ein Längenverhältnis der Geraden in einem Bereich von sechs bis zehn vorgesehen.

Das proximale Ende des Schaftteils ist als gerader, sich nach distal verjüngender Kegelstumpf ausgebildet, welcher an seinem distalen Ende in einen im wesentlichen zylindrisch ausgebildeten Schaftabschnitt absatzfrei übergeht. Der Durchmesser der proximalen Kreisfläche des Kegelstumpfes entspricht der Länge der großen Halbachse der elliptischen Gesamt-Querschnittsfläche am distalen Ende des Kopfteils. Er ist jedoch größer als die Länge der kleinen Halbachse der vorgenannten Querschnittsfläche. Der sich dadurch ergebende geringe Überstand des Schaftteils an der Trennstelle schafft bei implantierter Hüftgelenkprothese eine zusätzliche Verankerung des Schaftteils, welche in günstiger Weise einer Schaftlockerung entgegenwirkt, wenn ein medizinisch erforderlicher Austausch des Kopfteils durchgeführt werden soll.

Als Höhe des Kegelstumpfes ist ein Wert von einem Viertel bis einem Fünftel der wirksamen Schaftlänge günstig.

Entsprechend einer anderen Weiterbildung der Erfindung weist der zylindrische Abschnitt des Schaftteils eine Profilierung in Form von sich axial erstreckenden Rippen auf, deren periphere Kanten verrundet ausgebildet sind. Die Rippen sind gleichmäßig am Schaftumfang verteilt. Der zylindrisch ausgebildete Abschnitt des Schaftteils weist eine nach frontal gerichtete, gleichmäßige Krümmung auf und schafft somit in vorteilhafter Weise Voraussetzungen für die Anpassung der Prothese an die Anatomie des Oberschenkelknochens.

Entsprechend einer anderen vorteilhaften Ausführungsform der Erfindung weist das Schaftteil eine in Richtung der Schaftachse verlaufende Längsbohrung auf. Diese Längsbohrung läuft an ihrem distalen Ende in mindestens eine seitliche Öffnung in der Schaftwandung aus. Die seitliche Öffnung ist in Form eines Langloches ausgebildet. Sie dient einerseits dem Druckausgleich beim Einsetzen der Hüftgelenkprothese und ermöglicht andererseits in günstiger Weise den Austritt medizinischer Wirkstoffe, welche durch Plazieren eines Wirkstoffspenders am distalen Ende des Längsbohrung des Schaftteils einbringbar sind. Der mittlere Durchmesser der Längsbohrung weist deshalb einen Wert auf, welcher die Positionierung des Wirkstoffspenders in der Nähe der Wandungsöffnungen im distalen Bereich des Schaftteils ermöglicht.

Nach einer anderen Weiterbildung der Erfindung ist zwischen dem distalen Ende der Längsbohrung des Schaftteils und dem distalen Schaftende eine sich quer zur Schaftachse erstrekkende Durchgangsbohrung zur Aufnahme weiterer Fixierungsmittel vorgesehen. Der Durchmesser der Querbohrung ist derart gewählt, daß die Möglichkeit des Einsetzens eines Küntscher-Nagels besteht. Der Einsatz von Fixierungsmitteln am distalen Schaftende der Hüftgelenkprothese erhöht die Verdrehsicherheit und die axiale Belastbarkeit der Prothese.

Da die auf die Endoprothese wirkende Biegebelastung zeitlich nicht konstant ist, sonderen hinsichtlich Betrag und Richtung entsprechend den natürlichen Belastungszuständen der Endoprothese Schwankungen unterliegt, treten zwischen Zapfen und Bohrung Mikrobewegungen auf. Diese Mikrobewegungen können in Verbindung mit den am Mündungsrand der Bohrung auftretenden lokalen Spannungsspitzen zu einem Materialabtrag und damit zu einem frühzeitigen Verschleiß führen, was auch als Fretting bezeichnet wird.

Die am Mündungsrand der Bohrung auftretenden lokalen Spannungsspitzen rühren daher, daß bei einer weitgehend starren Zapfenaufnahme bei einer Verschränkung von Zapfen und Bohrung aufgrund einer Biegebelastung die wirksame spannungsaufnehmende Kontaktfläche verringert wird. Im Extremfall berührt der Zapfen die Innenwand der Bohrung nur noch unmittelbar am Mündungsrand sowie auf der gegenüberliegenden Seite am Boden der Bohrung.

Gemäß der Erfindung wird deshalb die Elastizität des Schaftelements kurz vor dem Mündungsende der Kegelbohrung erhöht, damit sich die Innenwand der Bohrung bei einer Biegebelastung der Endoprothese und damit zusammenhängend einer Verschränkung der Längsachsen von Zapfen und Bohrung an die Stellung des Zapfens anpaßt und mit der Mantelfläche des Zapfens eine hinreichend große spannungsaufnehmende Kontaktfläche bildet. Das unmittelbare Ende des Konus behält jedoch seine Wandungsstärke, um hier eine ausreichende Festigkeit beizubehalten. Somit wird erreicht, daß die Krafteinleitung im Endbereich des Konus über eine größere Länge verteilt wird und somit eine Überbeanspruchung im unmittelbaren Mündungsbereich vermieden ist.

Das Kopfteil bzw. das Schaftteil mit der konischen Bohrung weist hierzu an der Außenseite in Höhe der Bohrung eine in Bezug auf die Längsachse der Bohrung mindestens teilweise umlaufende Einkerbung auf. Hierdurch wird die Wandstärke der Zapfenaufnahme verringert und damit deren Nachgiebigkeit erhöht. Bei einer Biegebelastung der Gelenkprothese und daraus folgend einer Verschränkung von Zapfen und Bohrung gegeneinander paßt sich deshalb die Innenkontur der Bohrung an die Stellung des Zapfens an und die spannungsaufnehmende Kontaktfläche zwischen Zapfen und Bohrung wird nur geringfügig verringert, was in einer geringeren mechanische Belastung am Mündungsrand der Bohrung resultiert und damit zu einem geringeren mechanischen Verschleiß führt.

Durch die Schwächung des Materials infolge der Einkerbung erfolgt die Krafteinleitung nicht erst als maximale Spitze am Mündungsende der konischen Bohrung, sondern vergleichmäßigt auf der gesamten Kontaktfläche von Zapfen und Bohrung. Durch diese Vergleichmäßigung der Krafteinleitung wird der Maximalwert der mechanischen Spannung verringert und somit eine Überlastung des Materials verhindert.

Vorteilhafte Weiterbildungen der Erfindung sind in den Unteransprüchen gekennzeichnet bzw. werden nachstehend zusammen mit der Beschreibung der bevorzugten Ausführung der Erfindung anhand der Figuren näher dargestellt. Hierbei zeigen die Figuren 1 und 2 jedoch kein Ausführungsbeispiel der Erfindung, sondern erst die Figuren 5 bis 7. Es zeigen:

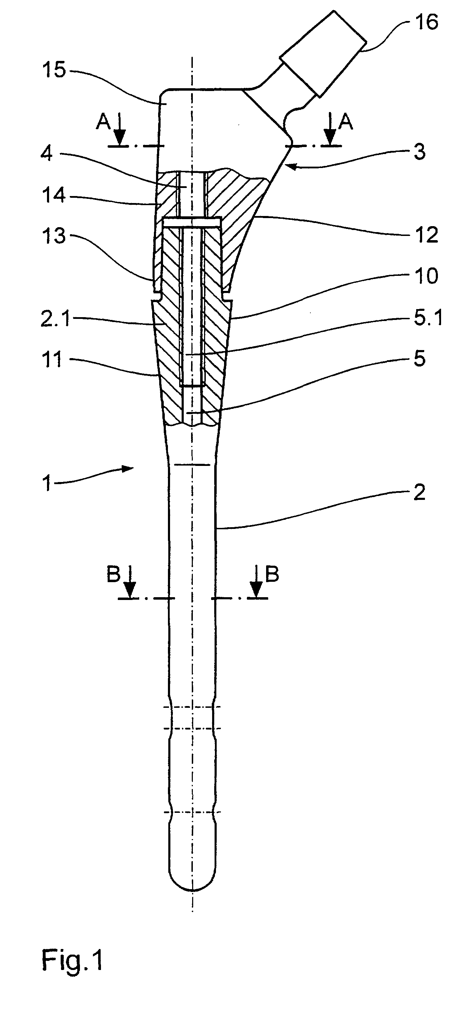

- Figur 1: eine modulare Hüftgelenkprothese als Darstellung eines Teillängsschnittes in Ansicht von dorsal bzw. frontal,

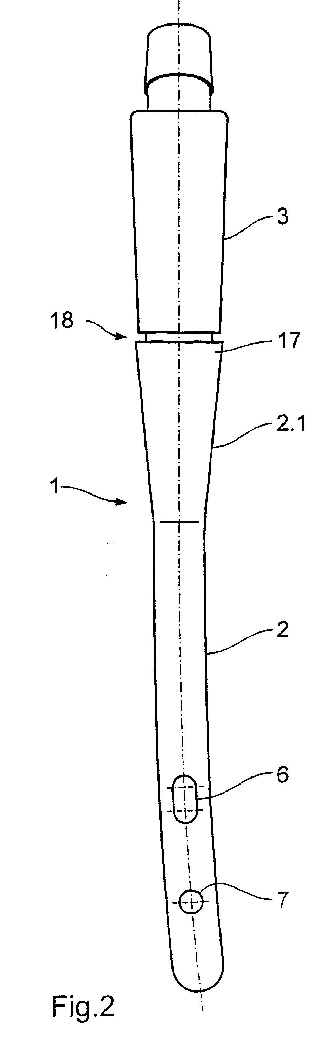

- Figur 2: die Darstellung der in Figur 1 gezeigten Ausführungsform der Erfindung in Ansicht von lateral,

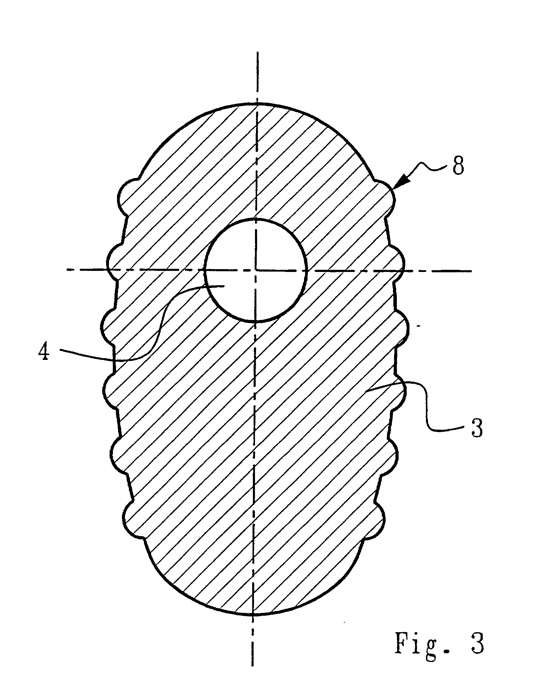

- Figur 3: eine Schnittdarstellung längs der Linie A...A gemäß Figur 1,

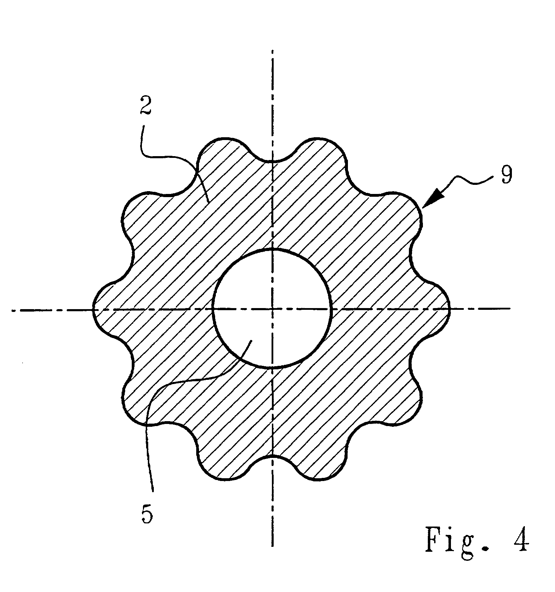

- Figur 4: einen weiteren Schnitt längs der Linie B...B gemäß Figur 1,

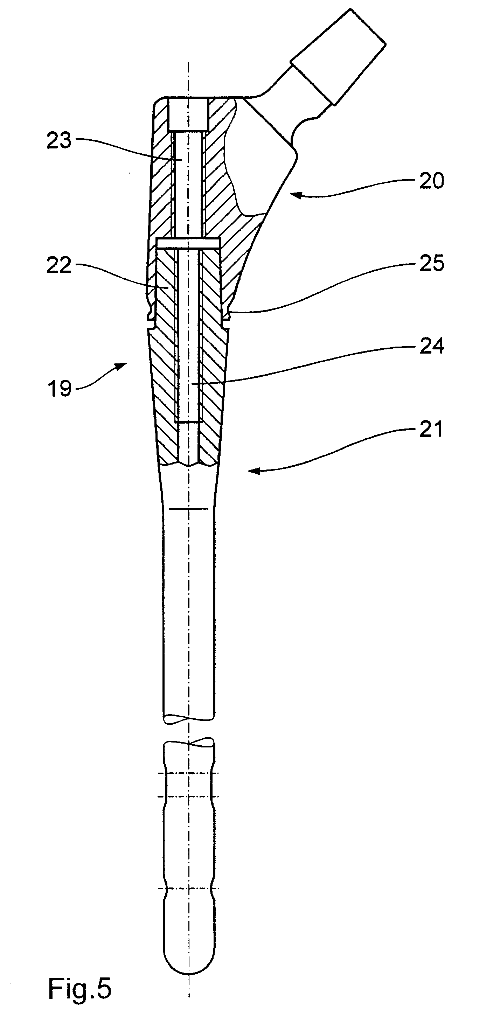

- Figur 5: eine Ausführungsform der Erfindung als Abwandlung der in Figur 1 gezeigten Gelenkprothese in Teilschnittdarstellung,

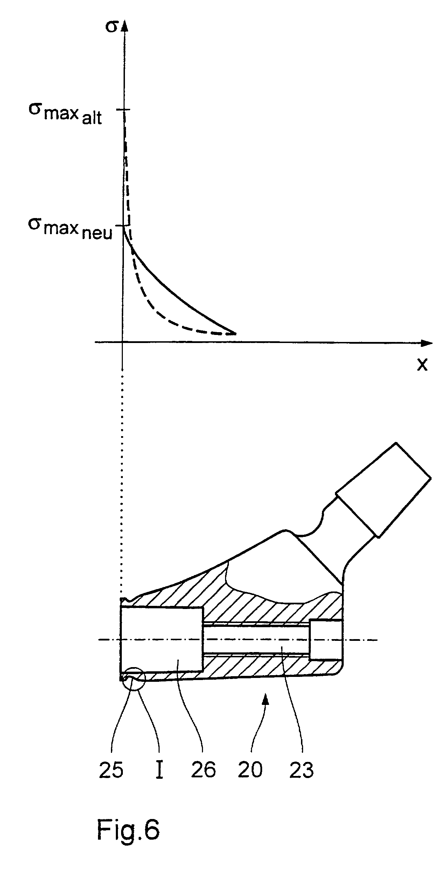

- Figur 6: das Kopfteil der in Figur 5 dargestellten Gelenkprothese ebenfalls in Teilschnittdarstellung sowie

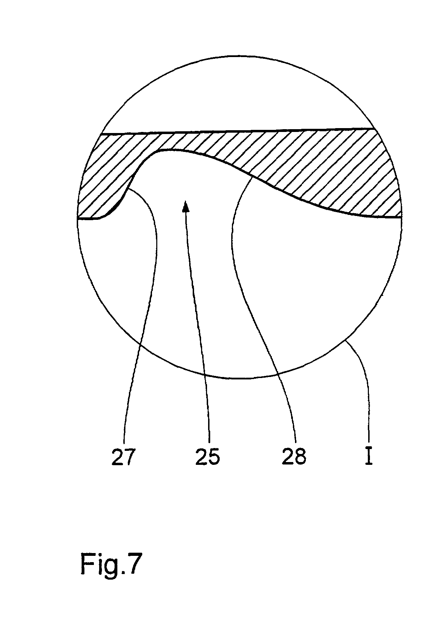

- Figur 7: einen Ausschnitt aus Figur 6.

In den Figuren 1 und 2 ist eine modulare Hüftgelenkprothese als Teillängsschnitt mit Ansicht von dorsal/frontal bzw. in Seitenansicht von lateral dargestellt. Die Hüftgelenkprothese 1 besteht aus einem Schaftteil 2 und einem Kopfteil 3, welche einem Bausatz für modulare Hüftgelenkprothesen entnommen sind, in dem im wesentlichen gleichartig ausgebildete Kopf- bzw. Schaftteile in verschiedenene Baugrößen vorgesehen sind. Die jeweils gewählten Einzelelemente 2, 3 sind vorzugsweise durch Zusammenstecken ihrer, entsprechende konische Zapfen oder Ausnehmung aufweisenden proximalen oder distalen Enden miteinander verbindbar. Die erforderliche Stabilität der Steckverbindung wird durch - vorzugsweise als Zuganker ausgebildete - Mittel (nicht dargestellt) gesichert. Das jeweilige Schaftteil 2 ist als Hohlschaft ausgebildet, wobei die sich axial erstreckende Längsbohrung 5 an ihrem proximalen Endabschnitt als Gewindebohrung 5.1 ausgebildet ist. Der (nicht dargestellte) Zuganker wird durch einen auf gleicher Achse wie die Längsbohrung 5 im Schaftteil 2 liegenden zylindrischen Kanal 4 im Kopfteil 3 geführt und in die Gewindebohrung 5.1 des Schaftteil 2 eingeschraubt.

Die Basis 15 des Kopfteils 3 weist bei Ansicht von dorsal bzw. frontal ein sich in proximaler Richtung erweiterndes Längsschnittprofil auf, das an seiner lateralen Seite durch zwei, einen stumpfen Winkel einschließende Geraden 13, 14 und an seiner medialen Seite durch ein konkaves Bogenstück 12 begrenzt ist. Dabei setzt sich die distal befindliche Gerade 13 des Geradenpaares 13, 14 und das Bogenstück 12 jeweils in einer Geraden 10, 11 fort, welche das sich nach distal konisch verjüngende Querschnittsprofil eines sich unterhalb des Einsteckkonus befindlichen proximalen, kegelstumpfförmigen Abschnitts 2.1 des Schaftteils 2 begrenzen.

Es ist ersichtlich, daß im Verbindungsbereich der Seitenansicht die Seitenlinien ohne wesentliche Steigungsänderung vom Schaftteil in den Kopfteil übergehen. Dies gilt im wesentlichen auch für die Ansicht der Schmalseite der Prothese, wie sie weiter unten beschrieben ist. Auf diese Weise kann zunächst einmal - bei gekrümmtem Schaftteil - ohne Verringerung der Kontinuität der Formkontur wahlweise eine links- oder eine rechtsseitige Prothese erzeugt werden. Da aber auch Zwischenstellungen ohne Schwierigkeiten bei ebenfalls gleichzeitiger Beibehaltung des optimierten Konturverlaufs eingestellt werden können, ist der Prothesensitz sehr präzise an die individuellen Gegebenheiten anpaßbar. Dies gilt auch für lange Schäfte, die als Knochennagelersatz in unterschiedlichen Längen zur Verfügung stehen. Damit ist mit einer minimalen Anzahl von Grundelementen die Versorgung einer Höchstzahl von durchaus unterschiedlich gelagerten Fällen möglich.

Die Geraden 13, 14 weisen eine unterschiedliche Länge und jeweils eine Neigung in Richtung der Mittelachse des Kopfteils 3 auf, wobei sich die kürzere Gerade 13 am distalen Ende des Kopfteils 3 befindet. Für das Längenverhältnis der Geraden 13, 14 ist ein Wert im Bereich von sechs bis zehn vorgesehen.

Das proximale Ende des Schaftteils 2 ist als gerader, sich nach distal verjüngender Kegelstumpf 2.1 ausgebildet, welcher an seinem distalen Ende in einen im wesentlichen zylindrisch ausgebildeten, eine kontinuierliche Krümmung nach frontal aufweisenden Schaftabschnitt absatzfrei übergeht. Eine derartige Formgebung am proximalen Schaftende sichert in vorteilhafter Weise einen festen Sitz des Schaftteils im Markraum des Oberschenkelknochens. Für das Höhenmaß des Kegelstumpfes 2.1 ist ein Viertel bis ein Fünftel der wirksamen Länge (d.h. des in den Markraum einzubringenden Schaftabschnitts) des Schaftteils 2 günstig. Der Durchmesser der proximalen Kreisfläche des Kegelstumpfes 2.1 entspricht der Länge der großen Halbachse des elliptischen Gesamt-Querschnittsfläche am distalen Ende des Kopfteils 3. Er ist jedoch größer als die Länge der kleinen Halbachse der vorgenannten Querschnittsfläche. Der sich dadurch ergebende geringe Überstand 17 des Schaftteils 2 an der Trennstelle 18 schafft bei implantierter Hüftgelenkprothese 1 eine zusätzliche Verankerung des Schaftteils 2, welche in günstiger Weise einer Schaftlockerung entgegenwirkt, wenn ein medizinisch erforderlicher Austausch des Kopfteils 3 durchgeführt werden soll.

Die Öffnung 6 in der Wandung des Schaftteils 2 bildet das distale Ende der Längsbohrung (vergleiche Position 5 gemäß Figur 1) des Schaftes der Hüftgelenkprothese 1. Sie ist als Langloch ausgebildet und dient einerseits dem Austritt von Wirkstoffen eines am Ende der Längsbohrung positionierten (nicht dargestellten) Wirkstoffspenders sowie andererseits dem Druckausgleich, wenn die Hüftgelenkprothese 1 mit ihrem Schaftteil 2 in den vorbereiteten Markraum eines Oberschenkelknochens eingebracht wird.

Die mit 7 bezeichnete Durchgangsbohrung am distalen Ende des Prothesenschaftes erstreckt sich quer zur Achse des Schaftteils 2. Diese Bohrung ist zur Aufnahme eines Fixationsmittels, beispielsweise eines Verriegelungs-Nagels, vorgesehen und in ihrem Durchmesser auf die möglichen Nagelabmessungen abgestimmmt. Die Verwendung zusätzlicher Fixationsmittel erhöht in vorteilhafter Weise die Verdrehsicherheit und die axiale Belastbarkeit einer implantierten Prothese.

Es ist ersichtlich, daß gemäß der Erfindung auch extreme Schaftlängen, für Anwendungsfälle, in denen bisher Nägel angewendet werden mußten, als Schaftprothesen vorgesehen sein können.

In den Figuren 3 und 4 ist jeweils ein Querschnittsprofil des Kopfteils 3 (Schnitt längs der Linie A...A gemäß Figur 1) und ein Querschnittsprofil des Schaftteils 2 (Schnitt läng der Linie B...B gemäß Figur 1) schematisch dargestellt. Die sich in axialer Richtung erstreckenden Rippen 8, 9 an den Breitseiten des Kopfteils 3 bzw. an der Peripherie des Schaftteils 2 sind peripher durch Kreisbögen begrenzt. Die Durchgangsbohrung in dem elliptischen Querschnittsprofils des Kopfteils 3 ist mit 4, die zentrale Längsbohrung des Schaftteils 2 mit 5 bezeichnet.

Die in Figur 5 in Querschnittsdarstellung wiedergegebene Gelenkprothese 19 ist eine Weiterbildung der in Figur 1 dargestellten Gelenkprothese, wobei die hier dargestellte Gelenkprothese 19 eine erhöhte mechanische Festigkeit bzw. einen geringeren Verschleiß aufweist und ein Ansführungsbeispiel der Erfindung zeigt.

Die dargestellte Gelenkprothese 19 besteht - wie die bereits in Figur 1 dargestellte Gelenkprothese - im wesentlichen aus einem Kopfteil 20 und einem mit diesem durch einen Konusanschluß verbundenen Schaftteil 21. Die mechanische Verbindung von Kopfteil 20 und Schaftteil 21 erfolgt also kraftschlüssig, indem ein an das Schaftteil 21 angeformter konischer Zapfen 22 in eine in dem Kopfteil 20 angeordnete konische Bohrung hineingesteckt wird und mit dieser eine Preßpassung bildet. Zur Verspannung von Kopfteil 20 und Schaftteil 21 gegeneinander dient ein Zuganker, der durch einen Kanal 23 in dem Kopfteil 20 hindurchgeführt und in eine Gewindebohrung 24 in dem Schaftteil 21 eingeschraubt wird.

Bei derartigen Gelenkprothesen besteht das Problem, daß bei einer Biebebelastung der Gelenkprothese am Mündungsrand der konischen Bohrung relativ große mechanische Spannungen auftreten. Die mechanischen Spannungsspitzen am Mündungsrand der konischen Bohrung rühren daher, daß bei einer Biegebelastung der Gelenkprothese 19 Zapfen 22 und Bohrung gegeneinander verschränkt werden, was zu einer Verringerung der wirksamen spannungsaufnehmenden Kontaktfläche zwischen Zapfen 22 und Bohrung führt.

Im Extremfall berührt der Zapfen 22 die Innenwand der Bohrung nur noch jeweils einseitig unmittelbar am Mündungsrand sowie auf der gegenüberliegenden Seite unmittelbar am Boden der Bohrung. Durch die Verringerung der wirksamen spannungsaufnehmenden Kontaktfläche entstehen deshalb insbesondere am Mündungsrand der Bohrung relativ große mechanische Spannungen.

Da die auf die Gelenkprothese 19 wirkende Biegebelastung in der Regel zeitlich nicht konstant ist, sondern hinsichtlich Betrag und Richtung entsprechend den natürlichen Belastungszuständen der Gelenkprothese 19 Schwankungen unterliegt, treten zwischen Zapfen 22 und Bohrung Mikrobewegungen auf. Diese Mikrobewegungen führen in Verbindung mit den am Mündungsrand der Bohrung auftretenden lokalen Spannungsspitzen zu einem Materialabtrag und damit zu einem frühzeitigen Verschleiß, was auch als Fretting bezeichnet wird.

Zur Verringerung dieser Abnutzungserscheinungen weist das Kopfteil 20 - im Gegensatz zu der in Figur 1 dargestellten Gelenkprothese - deshalb an der Außenwand nahe dem unteren Ende eine in Bezug auf die Längsachse der Bohrung umlaufende Einkerbung 25 auf. Durch diese Einkerbung 25 wird die Wandstärke des Kopfteils 20 verringert und damit die Nachgiebigkeit der Zapfenaufnahme gegenüber einer Verschränkung des Zapfens 22 erhöht. Wird der Zapfen 22 infolge einer Biegebeanspruchung der Gelenkprothese 19 gegenüber der Bohrung verschränkt, so gibt die Zapfenaufnahme - also die Innenkontur der Bohrung - dem Zapfen 22 nach und paßt sich der veränderten Stellung des Zapfens 22 an.

Durch diese elastische Anpassung der Zapfenaufnahme wird die wirksame spannungsaufnehmende Kontaktfläche zwischen Bohrung und Zapfen auch bei einer Biegebelastung der Gelenkprothese 19 nur unwesentlich verringert, was zu einer Verringerung der am Mündungsrand der Bohrung auftretenden mechanischen Belastung führt und den Verschleiß der Gelenkprothese 19 mindert.

Die in Figur 6 gezeigte Querschnittsdarstellung des Kopfteils 20 der in Figur 5 wiedergegebenen Gelenkprothese verdeutlicht die Form und die Anordnung der Einkerbung 25 in dem Kopfteil 20. So weist die Einkerbung 25 zunächst eine entlang der Längsachse des Kopfteils 20 zu derem Ende hin zunehmende Tiefe auf.

Einerseits wird dadurch erreicht, daß sich die Zapfenaufnahme - also die Innenkontur der Bohrung 26 - bei relativ geringen Biegebelastungen der Gelenkprothese und entsprechend geringen Verschränkungen von Zapfen und Bohrung 26 der veränderten Stellung des Zapfens gut anpaßt, was trotz der Verschränkung von Zapfen und Bohrung 26 zu einer relativ großen wirksamen spannungsaufnehmenden Kontaktfläche zwischen Zapfen und Bohrung 26 und damit zu einer Verringerung der mechanischen Belastung führt.

Andererseits wird durch die entlang der Längsachse des Kopfteils 20 nach oben hin abnehmende Nachgiebigkeit der Zapfenaufnahme sichergestellt, daß die Zapfenaufnahme - also die Innenkontur der Bohrung 26 - bei größeren Biegebelastungen der Gelenkprothese nur unwesentlich nachgibt, was für eine sichere, weitgehend spielfreie Führung des Zapfens unerläßlich ist. Die Zapfenaufnahme ist also bei relativ geringen Biegebelastungen relativ weich, was zu einer Verringerung der mechanischen Spannungen am Mündungsrand der Bohrung 26 führt, wird jedoch mit zunehmender Biegebeanspruchung härter, was einer sicheren Führung des Zapfens dient.

Die Einkerbung 25 führt einerseits zu einer Verringerung der mechanischen Spannung am Mündungsrand der Bohrung 26. Andererseits stellt die Einkerbung 25 jedoch eine mechanische Schwachstelle in dem Kopfteil 20 dar, die die Gefahr der Rißbildung und daraus folgend des mechanischen Versagens der Gelenkprothese in sich birgt. Um diese Gefahr zu verringern, weist die Einkerbung 25 eine glatte Form ohne hervorspringende oder einspringende Ecken oder Kanten auf. So läuft die Einkerbung 25 an deren oberem Ende glatt in der Außenwand des Kopfteils 20 aus, ohne einen Knick oder gar eine Stufe zu bilden. Hierdurch werden die in der Einkerbung 25 auftretenden Kerbspannungen und damit die Gefahr der Rißbildung verringert.

Weiterhin zeigt Figur 6 den Verlauf der in der Zapfenaufnahme auftretenden mechanischen Spannung entlang der Längsachse der Bohrung 26. Die gestrichelte Linie zeigt hierbei zum Vergleich den Spannungsverlauf bei der in Figur 1 dargestellten Gelenkprothese, während die durchgezogene Linie den Verlauf der mechanischen Spannung bei der vorstehend beschriebenen Gelenkprothese mit der Einkerbung 25 zeigt.

Bei der Gelenkprothese gemäß Figur 1 ist der Verlauf der mechanischen Spannung entlang der Längsachse der Bohrung 26 sehr stark nichtlinear. So ist die Spannung im oberen Bereich der Bohrung 26 relativ gering und nimmt in der Nähe des Mündungsrandes bis auf den Wert σ_{MAX,Alt} zu.

Bei der vorstehend beschriebenen Gelenkprothese ist der Spannungsverlauf entlang der Längsachse der Bohrung 26 dagegen wesentlich gleichmäßiger, was vorteilhaft in einer wesentlich geringeren Maximalspannug σ_{Max,Neu} resultiert.

Die Form der Einkerbung 25 ist detailliert aus Figur 7 ersichtlich, die den Ausschnitt I aus Figur 6 wiedergibt. Diese Darstellung verdeutlicht, daß die Einkerbung 25 unsymmetrisch ist und eine zum Ende des Zapfens hin zunehmende Tiefe aufweist. Die Einkerbung 25 weist also zwei Flanken 27, 28 unterschiedlicher Steigung auf, wobei die dem Zapfenende zugewandte Flanke 27 relativ steil verläuft und nur eine geringe Längserstreckung aufweist, während die dem Zapfenende abgewandte Flanke 28 relativ flach, aber langestreckt ist und in der Zapfenwand ausläuft.

Die Erfindung beschränkt sich in ihrer Ausführung nicht auf das vorstehend angegebene bevorzugte Ausführungsbeispiel. Vielmehr ist eine Anzahl von Varianten denkbar, welche von der dargestellten Lösung auch bei grundsätzlich anders gearteten Ausführungen Gebrauch macht.

## Patentansprüche

1. Modulare Hüftgelenkprothese (1, 19), welche aus einem mit einem Anschluß für eine Gelenkkugel versehenen Kopfteil (3) und einem Schaftteil (2) zusammenfügbar ist, wobei das Schaftteil (2) mit dem Kopfteil (3) mittels einer Steckverbindung mit diesem verbindbar ist und Mittel zur Arretierung der Steckverbindung vorgesehen sind, wobei die Steckverbindung im Bereich des Adamschen Bogens vorgesehen ist, wobei die Kontur von Kopf- und Schaftteil (2, 3) im Verbindungsbereich im Längsschnitt unabhängig von der relativen Ausrichtung der beiden Teile mit Ausnahme eines Spalts im unmittelbaren Anschlußbereich ohne wesentliche Richtungsänderung aneinander anschließt, und wobei
das Kopfteil (20) oder das Schaftteil (21) eine konische Bohrung als eine Zapfenaufnahme aufweist,
daß entsprechend das Schaftteil (21) oder das Kopfteil (20) einen passenden konischen Zapfen (22) aufweist
**dadurch gekennzeichnet, daß**
daß das Kopfteil (20) oder der Schaftteil (21) zu Verringerung der bei einer Biegebeanspruchung am Mündungsrand der Bohrung auftretenden mechanischen Belastung an der Außenwand in der Nähe des Mündungsendes eine umlaufende Einkerbung (25) aufweist.

2. Modulare Hüftgelenkprothese (1) nach Anspruch 1, **dadurch gekennzeichnet, daß** das Schaftteil (2) sich im Anschlußbereich zum Kopfteil (3) hin kegelstumpfartig erweitert.

3. Modulare Hüftgelenkprotheses (1) nach Anspruch 2, **dadurch gekennzeichnet, daß** die Neigung des sich kegelstumpfartig erweiternden Bereichs durch die Neigung der Kontur des Kopfteils (3) im Anschlußbereich fortgesetzt wird.

4. Modulare Hüftgelenkprothese (1) nach einem der vorangegangene Ansprüche, **dadurch gekennzeichnet, daß** das Kopfteil (3) ein sich in proximaler Richtung erweiterndes Längsschnittprofil bei Ansicht von dorsal/frontal aufweist, das an seiner lateralen Seite durch zwei, einen stumpfen Winkel einschließenden Geraden (13, 14) und an seiner medialen Seite durch einen konkaven Bogenbereich (12) begrenzt ist, wobei die distal befindliche Gerade (13) des Geradenpaares und das Bogenstück (12) sich jeweils in einer Geraden (10, 11) fortsetzen, welche das sich nach distal konisch verjüngende Längsschnittprofil eines sich unterhalb des Einsteckkonus befindlichen proximalen Abschnitts (2.1) des Schaftteils (2) begrenzen.

5. Modulare Hüftgelenkprothese (1) nach einem der vorangehenden Ansprüche, **dadurch gekennzeichnet, daß** die Basis (15) des Kopfteils (3) in allen Ebenen ein im wesentlichen elliptisches Querschnittsprofil aufweist.

6. Modulare Hüftgelenkprothese (1) nach Anspruch 5, **dadurch gekennzeichnet, daß** die große Halbachse der Ouerschnitts-Ellipse nach distal kontinuierlich abnimmt.

7. Modulare Hüftgelenkprothese (1) nach Anspruch 1, **dadurch gekennzeichnet, daß** das proximale Ende des Schaftteils (2) unterhalb des Einsteckkonus als Kegelstumpf (2.1) ausgebildet ist, welcher sich nach distal bis auf das Durchmesser-Endmaß des Schaftes verjüngt.

8. Modulare Hüftgelenkprothese (1) nach Anspruch 7, **dadurch gekennzeichnet, daß** die Höhe des Kegelstumpfes (2.1) einem Viertel bis einem Fünftel der wirksamen Schaftlänge entspricht.

9. Modulare Hüftgelenkprothese (1) nach Anspruch 8, **dadurch gekennzeichnet, daß** der sich an das distale Ende des Kegelstumpfes (2.1) anschließende Abschnitt des Schaftteils (2) eine gleichmäßige Krümmung nach frontal aufweist.

10. Modulare Hüftgelenkprothese (1) nach Anspruch 1, **dadurch gekennzeichnet, daß** das Schaftteil (2) eine in Richtung der Schaftachse verlaufende Längsbohrung (5) aufweist.

11. Modulare Hüftgelenkprothese (1) nach Anspruch 10, **dadurch gekennzeichnet, daß** die Längsbohrung (5) an ihrem distalen Ende in mindestens eine seitliche Öffnung (6) in der Schaftwandung ausläuft.

12. Modulare Hüftgelenkprothese (1) nach Anspruch 11, **dadurch gekennzeichnet, daß** die seitliche Öffnung (6) die Form eines Langloches aufweist.

13. Modulare Hüftgelenkprothese (1) nach einem der Ansprüche 9 bis 12, **dadurch gekennzeichnet, daß** zwischen dem distalen Ende der Längsbohrung (5) und dem distalen Ende des Schaftteils (2) eine Querbohrung (7) zur Aufnahme eines zusätzlichen Fixierungsmittels vorgesehen ist.

14. Modulare Hüftgelenkprothese (1) nach einem der vorangehenden Ansprüche, **dadurch gekennzeichnet, daß** an den Breitseiten der Basis (15) des Kopfteils (2) eine Profilierung aus sich axial erstreckenden, verrundete Außenkanten aufweisenden Rippen (8) vorgesehen ist.

15. Modulare Hüftgelenkprothese (1) nach einem der **vorangehenden Ansprüche, dadurch gekennzeichnet, daß** das Schaftteil (2) in seinem gekrümmt ausgebildeten Abschnitt eine Längsprofihierung aus verrundete Kanten aufweisenden Rippen (9) aufweist.

16. Modulare Hüftgelenkprothese (1) nach Anspruch 15, **dadurch gekennzeichnet, daß** die Rippen (9) gleichmäßig am Schaftumfang verteilt angeordnet sind.

17. Modulare Hüftgelenkprothese (1) nach einem der vorhergehenden Ansprüche, **dadurch gekennzeichnet, daß** die Tiefe der Einkerbung (25) mindestens teilweise entlang der Längsachse der Bohrung zum distalen Ende des Kopfteils (20) hin zunimmt.

## Claims

1. Modular hip joint prosthesis (1, 19) which can be formed from a head part (3) having a connector for a ball joint and from a shaft part (2) wherein the shaft part (2) is connectable with the head part (3) by a push-fit connection and means are provided to lock the push-fit connection, wherein the push-fit connection is provided in the region of the Adams curve and the contours of the head and shaft parts (2, 3) in the connecting area in longitudinal section independently of the relative alignment of the two parts except for a gap in the immediate joining area follow on from one another without any significant change in direction, and
wherein the head part (20) or shaft part (21) has a conical bore as a stud socket and
the shaft part (21) or the head part (20) correspondingly has a snug-fitting conical stud (22),
**characterised in that**
the head part (20) or shaft part (21) has a circumferential indented area (25) around the outside wall close to the mouth end in order to reduce the mechanical stress which arises in the event of bending strain on the edge of the mouth of the bore.

2. Modular hip joint prosthesis (1) according to claim 1 **characterised in that** the shaft part (2) widens out in the joining area frusto-conically towards the head part (3).

3. Modular hip joint prosthesis (1) according to claim 2 **characterised in that** the incline of the frusto-conically widening area is continued in the joining area through the incline of the contour of the head part (3).

4. Modular hip joint prosthesis (1) according to one of the preceding claims **characterised in that** the head part (3) has a longitudinal sectional profile widening out in the proximal direction in a dorsal/frontal view which is restricted on its lateral side through two straight lines (13, 14) which include an obtuse angle, and on its medial side through a concave arcuate area (12), wherein the distal straight line (13) of the pair of straight lines and the arcuate piece (12) each continue in a straight line (10, 11) which together define the distal conically tapering longitudinal sectional profile of a proximal section (2.1) of the shaft part (2) located below the insert cone.

5. Modular hip joint prosthesis (1) according to one of the preceding claims, **characterised in that** the base (15) of the head part (3) has a substantially elliptical cross-sectional profile in all planes.

6. Modular hip joint prosthesis (1) according to claim 5 **characterised in that** the large semi-axis of the cross-sectional ellipse decreases continuously towards the distal side.

7. Modular hip joint prosthesis (1) according to claim 1 **characterised in that** the proximal end of the shaft part (2) below the insert cone is formed as a truncated cone (2.1) which tapers towards the distal end down to the final end diameter size of the shaft.

8. Modular hip joint prosthesis (1) according to claim 7 **characterised in that** the height of the truncated cone (2.1) corresponds to a quarter to a fifth of the effective shaft length.

9. Modular hip joint prosthesis (1) according to claim 8 **characterised in that** the section of the shaft part (2) adjoining the distal end of the truncated cone (2.1) has a uniform curvature to the frontal side.

10. Modular hip joint prosthesis (1) according to claim 1 **characterised in that** the shaft part (2) has a longitudinal bore (5) running in the direction of the shaft axis.

11. Modular hip joint prosthesis (1) according to claim 10 **characterised in that** the longitudinal bore (5) opens out at its distal end into at least a lateral opening (6) in the shaft wall.

12. Modular hip joint prosthesis (1) according to claim 11 **characterised in that** the lateral opening (6) has the shape of an elongated hole.

13. Modular hip joint prosthesis (1) according to one of claims 9 to 12 **characterised in that** a cross bore (7) for holding additional fixing means is provided between the distal end of the longitudinal bore (5) and the distal end of the shaft part (2).

14. Modular hip joint prosthesis (1) according to one of the preceding claims **characterised in that** a profiled area of axially extending ribs (8) having rounded outside edges is provided on the broad sides of the base (15) of the head part (2).

15. Modular hip joint prosthesis (1) according to one of the preceding claims **characterised in that** the shaft part (2) has in its curved section a longitudinal profiled area of ribs (9) having rounded edges.

16. Modular hip joint prosthesis (1) according to claim 15 **characterised in that** the ribs (9) are arranged spaced out uniformly round the circumference of the shaft.

17. Modular hip joint prosthesis (1) according to one of the preceding claims **characterised in that** the depth of the indented area (25) increases at least in part along the longitudinal axis of the bore towards the distal end of the head part (20).

## Revendications

1. Prothèse modulaire (1, 19) de l'articulation de la hanche, qui peut être formée par l'assemblage d'une partie de tête (3) comportant un raccord pour une articulation sphérique, et une partie formant tige (2), et dans laquelle la partie formant tige (2) peut être reliée à la partie de tête (3) au moyen d'une liaison à enfichage, et dans laquelle des moyens sont prévus pour bloquer la liaison à enfichage, et dans laquelle la liaison à enfichage est prévue au niveau du coude d'Adam, les contours de la partie de tête et de la partie formant tige (2, 3) se raccordent entre eux dans la zone de liaison en coupe longitudinale, indépendamment de l'orientation relative des deux parties, à l'exception d'une fente dans la zone de raccordement direct sans modification sensible de l'orientation, et
dans laquelle la partie de tête (20) ou la partie formant tige (21) possède un perçage conique, qui est utilisé en tant que logement pour un embout et qui possède, conformément à la partie formant tige (21) ou la partie de tête (20), un embout conique adapté (22),
**caractérisée en ce**
**que** la partie de tête (20) ou la partie formant tige (21) possède une encoche périphérique (26) pour réduire la contrainte mécanique, qui apparaît lors d'une contrainte de flexion au niveau du bord de l'embouchure du perçage, au niveau de la paroi extérieure à proximité de l'extrémité de l'embouchure.

2. Prothèse modulaire (1) de l'articulation de la hanche selon la revendication 1, **caractérisée en ce que** la partie formant tige (2) s'élargit avec une forme tronconique dans la zone de raccordement en direction de la partie de tête (3).

3. Prothèse modulaire (1) de l'articulation de la hanche selon la revendication 2, **caractérisée en ce que** l'inclinaison de la partie, qui s'élargit avec une forme tronconique, se prolonge dans la zone de raccordement par l'inclinaison du contour de la partie de tête (3).

4. Prothèse modulaire (1) de l'articulation de la hanche selon l'une des revendications précédentes, **caractérisée en ce que** la partie de tête (3) comporte selon une vue dorsale/frontale, un profil en coupe longitudinale qui s'étend dans la direction proximale et qui est limité, sur son côté latéral, par deux droites (13, 14) qui font entre elles un angle obtus et, sur son côté médial, par une zone d'arc concave (12), la droite (13), située sur le côté distal, du couple de droites et l'élément d'arc (12) se prolongeant respectivement par des droites (10, 11), qui délimitent le profil en coupe longitudinale, qui se rétrécit avec une forme conique sur le côté distal, d'une partie proximale (2.1) d'une section proximale (2.1) de la partie formant tige (2), qui est située au-dessous du cône d'enfichage.

5. Prothèse modulaire (1) de l'articulation de la hanche selon l'une des revendications précédentes, **caractérisée en ce que** la base (15) de la partie de tête (3) possède dans tous les plans, un profil en coupe transversale sensiblement elliptique.

6. Prothèse modulaire (1) de l'articulation de la hanche selon la revendication 5, **caractérisée en ce que** le demi grand axe de l'ellipse en coupe transversale diminue continûment sur le côté distal.

7. Prothèse modulaire (1) de l'articulation de la hanche selon la revendication 1, **caractérisée en ce que** l'extrémité proximale de la partie formant tige (2) est formée au-dessous du cône d'enfichage en tant qu'embout conique (2.1), qui se rétrécit sur le côté distal, jusqu'à la cote finale du diamètre de la tige.

8. Prothèse modulaire (1) de l'articulation de la hanche selon la revendication 7, **caractérisée en ce que** la hauteur du tronc de cône (2.1) correspond à une valeur comprise entre 1/4 et 1/5 de la longueur efficace de la tige.

9. Prothèse modulaire (1) de l'articulation de la hanche selon la revendication 8, **caractérisée en ce que** la section de la partie formant tige (2), qui se raccorde à l'extrémité distale du tronc de cône (2.1) possède sur le côte frontal une courbure uniforme.

10. Prothèse modulaire (1) de l'articulation de la hanche selon la revendication 1, **caractérisée en ce que** la partie formant tige (2) possède un perçage longitudinal (5) qui s'étend dans la direction de l'axe de la tige.

11. Prothèse modulaire (1) de l'articulation de la hanche selon la revendication 10, **caractérisée en ce que** le perçage longitudinal (5) se termine au niveau de son extrémité distale, par au moins une ouverture latérale (6) formée dans la paroi de la tige.

12. Prothèse modulaire (1) de l'articulation de la hanche selon la revendication 1, **caractérisée en ce que** l'ouverture latérale (6) possède la forme d'un trou allongé.

13. Prothèse modulaire (1) de l'articulation de la hanche selon l'une des revendications 9 à 12, **caractérisée en ce qu'**un perçage transversal (7) servant à recevoir un moyen de fixation supplémentaire est prévu entre l'extrémité distale du perçage allongé (5) et l'extrémité distale de la partie formant tige (2).

14. Prothèse modulaire (1) de l'articulation de la hanche selon l'une des revendications précédentes, **caractérisée en ce qu'**un profil formé par des nervures axiales (8), qui possèdent des bords extérieurs arrondis, est prévu sur les côtés larges de la base (15) de la partie de tête (2).

15. Prothèse modulaire (1) de l'articulation de la hanche selon l'une des revendications précédentes, **caractérisée en ce que** la partie formant tige (2) comporte, dans sa section agencée avec une forme cintrée, un profil longitudinal constitué par les nervures (9) possédant des bords arrondis.

16. Prothèse modulaire (1) de l'articulation de la hanche selon la revendication 15, **caractérisée en ce que** les nervures (9) sont disposées en étant réparties uniformément sur le pourtour de la tige.

17. Prothèse modulaire (1) de l'articulation de la hanche selon l'une des revendications précédentes, **caractérisée en ce que** la profondeur de l'encoche (25) augmente au moins partiellement le long de l'axe longitudinal du perçage en direction de l'extrémité distale de la partie de tête (20).
